# FASCICULE DE BREVET EUROPEEN

(11) **EP 2 838 399 B1**
(45) Date de publication et mention de la délivrance du brevet: **08.06.2016**
(21) Numéro de dépôt: 13725391.0
(22) Date de dépôt: 16.04.2013
(51) Int. Cl.: A45D 34/04, B05B 11/00, B65D 83/28

(54) **TÈTE DE DISTRIBUTION ET D'APPLICATION**
VERTEILUNGS- UND APPLIKATIONSKOPF
DISTRIBUTION AND APPLICATION HEAD

(30) Priorité: 20.04.2012 FR 1253630
(43) Date de publication de la demande: 25.02.2015
(73) Titulaire: Aptar France SAS, 27110 Le Neubourg (FR)
(72) Inventeur: MOREAU, Francis, 76300 Sotteville Les Rouen (FR)
(74) Mandataire: CAPRI
(86) Numéro de dépôt international: PCT/FR2013/050834
(87) Numéro de publication internationale: WO 2013/156730

(56) Documents cités:
- EP-A2- 1 293 448
- WO-A2-2010/141159
- FR-A1- 2 954 936

## Description

La présente invention concerne une tête de distribution et d'application destinée à être associée à une unité de distribution, telle qu'une pompe, une valve, un tube souple, etc. La tête comprend un patin d'application définissant une face extérieure d'application destinée à venir en contact de la peau, le patin étant réalisé essentiellement en un matériau caloporteur pour conférer une sensation de froid au contact de la peau, le patin définissant une épaisseur à partir d'une face intérieure vers la face extérieure, le patin étant traversé par un passage de distribution faisant communiquer les deux faces intérieure et extérieure. Les domaines d'application privilégiés de la présente invention sont ceux de la cosmétique, de la pharmacie ou encore de la parfumerie.

Dans le domaine de la cosmétique et de la parfumerie, il existe déjà des distributeurs de produit fluide comprenant un réservoir de produit fluide associé à une tête de distribution rigide et caloporteuse, destinée à être mise en contact avec la peau, notamment celle du visage. On connaît par exemple les documents WO2010141158, WO2010141159, FR2954936 et FR2915972. La tête forme un patin d'application au niveau duquel débouche un orifice de distribution. L'utilisateur actionne le distributeur pour émettre une dose de produit fluide, puis l'étale à l'aide du patin d'application, qui procure un effet massant et surtout une sensation de froid au contact de la peau, ce qui est fort agréable.

Dans le document FR2915972, le patin en métal ou céramique définit un alésage dans lequel est reçu un insert plastique qui forme un conduit de sortie et l'orifice de distribution. De ce fait, le produit fluide distribué n'est jamais en contact de l'alésage, mais l'orifice est visiblement formé par une pièce (l'insert) qui est distincte du patin, ce qui n'est pas vraiment esthétique.

Dans les documents WO2010141158 et WO2010141159, le patin comprend un passage de distribution tronconique définissant sur sa face intérieure une entrée très large et sur la face extérieure un orifice de distribution. Le produit fluide distribué vient en contact de la totalité du passage tronconique, et se refroidit à son contact. On peut également dire que le produit fluide réchauffe le patin du fait du contact étendu. Dans la présente invention, le produit fluide vient en contact du passage de distribution, comme dans les documents WO2010141158 et WO2010141159 susmentionnés.

La présente invention cherche à préserver les avantages de ces patins de l'art antérieur, tout en éliminant leurs inconvénients. Le produit fluide doit directement traverser le patin (sans insert), sans pour autant le réchauffer. Un autre but de la présente invention est de faciliter un éventuel traitement de surface du patin, notamment au niveau du passage de distribution.

Pour ce faire, la présente invention propose une tête de distribution et d'application destinée à être associée à une unité de distribution, telle qu'une pompe, une valve ou un tube souple, la tête comprenant un patin d'application définissant une face extérieure d'application destinée à venir en contact de la peau, le patin étant réalisé essentiellement en un matériau caloporteur pour conférer une sensation de froid au contact de la peau, le patin définissant une épaisseur à partir d'une face intérieure vers la face extérieure, le patin étant traversé par un passage de distribution faisant communiquer les deux faces intérieure et extérieure, le patin d'application étant réalisé en métal, tel que du zamak, et recouvert d'une couche protectrice barrière matériau inerte, tel que du nickel et/ou du chrome, au moins au niveau du passage de distribution et de la surface d'application, la tête comprenant en outre un corps en matière plastique définissant une section de raccordement à l'unité de distribution, une section d'application en prise avec la face intérieure du patin d'application, et une section de liaison reliant la section de raccordement à la section d'application, le corps définissant un canal de distribution qui communique avec le passage de distribution du côté de la face intérieure, le patin d'application présentant l'épaisseur minimum au niveau du passage de l'ordre de 1 à 4 mm.

Ainsi, le produit fluide traverse directement le patin en passant à travers le passage de distribution, et ne subit qu'un faible réchauffement du fait que le passage de distribution est très court. Pour l'utilisateur, le produit fluide sort directement du patin (et non pas d'un insert plastique), et le patin reste « froid », puisqu'il n'est que très peu influencé par le produit fluide.

La face intérieure peut par exemple présenter une concavité supérieure à la convexité de la face extérieure.

La faible profondeur du passage de distribution permet d'appliquer un revêtement sur le patin sans risquer une imperfection au niveau du passage. En effet, si le passage est long et fin, il est extrêmement difficile de garantir une parfaite application du revêtement dans le passage. Un tel revêtement est notamment nécessaire avec un patin en zamak, qui n'est pas inerte pour certains produits fluides, comme les crèmes cosmétiques : il faut donc lui appliquer un revêtement inerte protecteur ou barrière.

Avantageusement, la section de liaison est souple de sorte que le patin d'application est déplaçable par rapport à la section de raccordement. Avantageusement, la section d'application est surmoulée sur le patin d'application.

Selon une autre caractéristique, la section d'application est en contact de la majeure partie de la face intérieure du patin d'application. Avantageusement, le canal de distribution comprend une extrémité aval qui est directement adjacent au passage de distribution. De préférence, le canal de distribution définit une cavité sensiblement sphérique immédiatement en amont du passage de distribution.

Un des principes de la présente invention réside dans le fait de former un passage de distribution très court dans le patin, le produit fluide étant directement alimenté dans le passage, de préférence sans autre contact préalable avec le patin. Un tel patin peut aisément être revêtu d'une couche inerte, si nécessaire.

Sur les figures :
La figure 1 est une vue en section transversale verticale à travers un distributeur de produit fluide selon un mode de réalisation de l'invention,
La figure 2 est une vue en plan du distributeur de la figure 1a,
La figure 3 est une vue agrandie en en section transversale verticale à travers la tête distribution des figures 1 et 2.

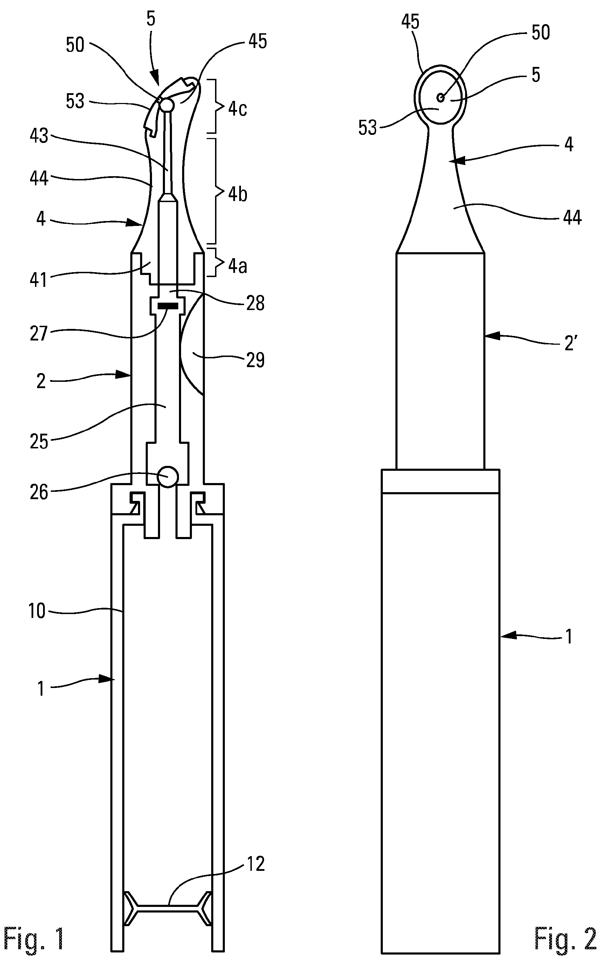

On se référera tout d'abord aux figures 1 et 2 pour décrire en détail la structure du distributeur de produit fluide selon le premier mode de réalisation de l'invention. Le distributeur comprend essentiellement cinq éléments constitutifs, à savoir un réservoir de produit fluide 1, une unité de distribution 2 qui est ici une pompe à actionnement latéral, et une tête de distribution 3 constituée d'une pièce de raccordement et de liaison 4 associée à un patin d'application 5.

Le réservoir de produit fluide 1 peut être de toute nature, de toute forme et réalisée dans des matériaux divers appropriés. Dans le domaine de la cosmétique, il est fréquent d'utiliser un réservoir particulier tel que représenté sur la figure 1, comprenant un fût de coulissement cylindrique 10 associé à un piston suiveur 12 qui se déplace dans le fût 10 à mesure que du produit fluide en a été extrait. Ce type de réservoir permet de conserver le produit fluide hors de contact de l'air extérieur. Le piston suiveur 12 est disposé à une extrémité du fût 10 lorsque le réservoir est rempli, qui est opposé à l'autre extrémité du fût formant un col 11 définissant une ouverture faisant communiquer l'intérieur du réservoir avec l'extérieur. Il s'agit là d'une conception tout à fait classique pour un réservoir du type piston suiveur.

La pompe 2 est une pompe à actionnement latéral 2 montée sur le réservoir 1. La pompe 2 comprend un clapet d'entrée 26 et un clapet de sortie 27 entre lesquels est formée une chambre de pompe 25 comprenant une paroi d'actionnement latéral 29 permettant de réduire le volume interne de la chambre 25. Il est à noter que la sortie 28 de la pompe 2 est fixe et située dans l'axe de la pompe et du distributeur. La tête 3 est montée sur la sortie 28 de la pompe 2 de sorte qu'elle ne subit aucun déplacement lors de l'actionnement de la pompe 2 par le poussoir latéral 29.

La tête de distribution et d'application 3 est montée sur la sortie 28 de la pompe 2 et comprend un conduit d'alimentation interne 43 qui relie la sortie 28 à un passage de distribution 50. Comme déjà mentionné, la tête de distribution et d'application 3 est constituée d'une pièce de raccordement et de liaison 4 associée à un patin d'application 5. La pièce de raccordement et de liaison 4 est réalisée en matière plastique, alors que le patin d'application 5 est réalisé en un matériau caloporteur dur ou rigide, tel que du métal, de la céramique, un matériau minéral etc., apte à conférer une sensation de froid au contact de la peau. De préférence, le patin 5 est réalisé en zamak revêtu d'une couche inerte protectrice barrière, tel que du nickel et/ou du chrome.

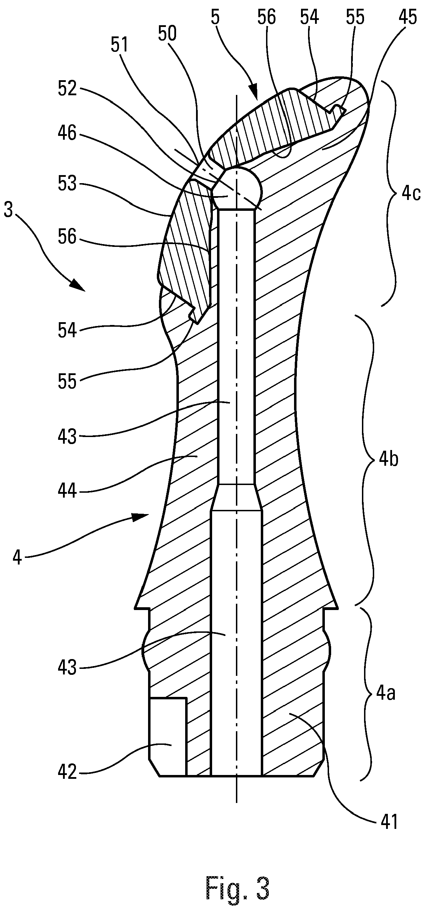

En se référant à la figure 3, on peut voir la structure détaillée la tête de distribution et d'application 3. Le patin d'application 5 est une pièce monobloc massive qui présent de préférence une symétrie de révolution. Il définit une face extérieure d'application 53 et une face intérieure 56 qui en contact intime avec la pièce de raccordement et de liaison 4. Sur sa périphérie externe, le patin 5 définit un chant annulaire 54 qui peut être formé avec un cordon saillant d'accrochage 55. Ce cordon 55 peut être adjacent à la face intérieure 53. Le patin 5 est traversé par un passage de distribution 50 qui relie les deux faces 53, 56. Sur la face extérieure 53, le passage de distribution 50 définit un orifice de distribution 51, et sur la face intérieure 56, le passage de distribution 50 définit un orifice d'entrée 52. Le passage de distribution 50 est avantageusement situé au centre du patin 5, mais une disposition excentrée peut également être envisagée. Le passage de distribution 50 définit un axe de distribution qui s'étend obliquement vers le haut par rapport à l'axe longitudinal de la tête 3.

La face extérieure d'application 53 est convexe ou bombée : sa fonction est de recevoir une dose de produit fluide et de l'étaler ou l'appliquer sur une surface d'application, telle que la peau du visage. La face intérieure 56 est concave, par exemple de forme globalement tronconique. On peut remarquer que la concavité de la face intérieure est supérieure à la convexité de la face extérieure 53, de sorte que l'épaisseur du patin est plus grande au niveau de sa périphérie externe qu'au niveau de son centre, où est avantageusement situé le passage de distribution 50. En d'autres termes, le patin 5 présente une épaisseur minimum au niveau du passage de distribution 50. On peut ainsi dire que le passage de distribution 50 est peu profond ou court, par exemple de l'ordre de 1 à 4 mm. Son diamètre peut également être de l'ordre de 1 à 4 mm, de sorte que le rapport diamètre/profondeur peut être de l'ordre de 1, comme c'est environ le cas sur la figure 3. Avec un passage de distribution 50 ayant une telle configuration, il est facile d'appliquer un revêtement inerte sur la paroi du passage, ce qui ne serait pas le cas avec un passage long ou profond.

Comme susmentionné, le patin d'application5 est associé, de préférence par surmoulage, avec la pièce de raccordement et de liaison 4 pour former ensemble la tête de distribution et d'application 3. La pièce de raccordement et de liaison 4 définit 3 section distinctes, à savoir une section de raccordement 4a, une section de liaison 4b et une section d'application 4c conjointement avec le patin d'application 5. Plus précisément, la matière plastique constitutive de la pièce 4 est surmoulée sur le patin 5, de manière à former une sorte de coque surmoulée 45. La coque surmoulée 45 s'étend autour de du chant 54 et du cordon 55, et sur presque toute la surface de face intérieure 56. On peut remarquer que la section 4c forme directement en amont du passage de distribution 50, c'est-à-dire en amont de l'orifice d'entrée 52, une cavité partiellement ou sensiblement sphérique 46 dont le diamètre est supérieur à celui du le passage de distribution 50. La cavité 46 est largement ouverte sur le passage de distribution 50 à travers l'orifice d'entrée 52, vient en contact annulaire avec la partie de la face intérieure 56 qui borde l'orifice d'entrée 52. La cavité 46 se prolonge en amont par un canal de distribution 43 qui s'étend à travers les deux autres sections 4b et 4a. Lors du surmoulage de la pièce 4 sur le patin 5, le canal 43 et la cavité 46 sont formés par une broche de moulage allongée qui comprend à son extrémité libre une boule sphérique correspondant à la cavité. Du fait de sa forme sphérique, la boule peut aisément et assurément venir en contact intime étanche annulaire avec le bord de l'orifice d'entrée 52 du patin 5. Lors du démoulage, la brioche est extraite de la pièce 4 en force, notamment lorsque la boule présente un diamètre supérieur à celui du canal, comme c'est le cas sur la figure 3.

La section de liaison 4b, qui se raccorde en amont de la section d'application 4c, forme un tube de liaison 44 définissant intérieurement le canal de distribution 43. Avantageusement, la section de liaison 4b, au niveau de son tube de liaison 43, présente une déformabilité élastique. Cette déformabilité est conférée par les qualités intrinsèques des matières plastiques utilisées et/ou des épaisseurs de paroi réduites à ce niveau. La section de liaison 4b se prolonge à son extrémité inférieure par la section de raccordement 4a qui comprend un manchon de raccordement 41 emmanché à la sortie 28 de la pompe. Le manchon 41 peut intégrer un profil d'orientation 42 qui coopère avec un profil complémentaire de la pompe pour orienter angulairement la tête 3 par rapport à la pompe 2. La section de raccordement 4a présente une rigidité supérieure à celle de la section de liaison 4b, principalement du fait des épaisseurs de paroi accrues.

La tête de distribution et d'application 3 associe un patin d'application caloporteur et de sensation froide à une pièce de raccordement et de liaison 4. Une fois en place sur la pompe 2, l'utilisateur peut appuyer sur le poussoir 29 pour distribuer une dose de produit fluide au niveau de l'orifice de distribution 51. Ensuite, il peut appliquer le patin sur une surface d'application souhaitée, telle que la peau, et étaler le produit fluide en déplaçant le patin sur la peau, lui procurant ainsi une sensation de massage froid. Les mouvements de la main imprimés au niveau du réservoir 1 sont transmis au patin avec un effet atténué ou amorti, du fait de l'élasticité de la section de liaison 4b.

Sans sortir du cadre de l'invention, le patin 5 pourrait être asymétrique, le passage de distribution 50 pourrait être excentré, le passage de distribution 50 pourrait s'étendre dans l'axe du canal de distribution 43 ou perpendiculairement à celui-ci, la coque 45 pourrait ne s'étendre que sur une partie de la face intérieure 56, la coque 45 pourrait ne pas s'étendre sur le chant 54, la tête 3 pourrait être montée à la sortie d'une pompe classique à actionnement axial, d'une valve ou encore d'un tube souple écrasable.

La profondeur réduite du passage de distribution 50 permet au produit fluide de traverser directement le patin sans pour autant le perturber thermiquement. De plus, lorsque le patin est réalisé en un matériau qui n'est pas inerte, tel que du zamak, il est possible d'appliquer un revêtement inerte même au niveau du passage de distribution 50, du fait de sa profondeur réduite.

## Revendications

1. Tête de distribution et d'application (3) destinée à être associée à une unité de distribution (2), telle qu'une pompe, une valve ou un tube souple, la tête comprenant un patin d'application (5) définissant une face extérieure d'application (53) destinée à venir en contact de la peau, le patin (5) étant réalisé essentiellement en un matériau caloporteur pour conférer une sensation de froid au contact de la peau, le patin (5) définissant une épaisseur à partir d'une face intérieure (56) vers la face extérieure (53), le patin (5) étant traversé par un passage de distribution (50) faisant communiquer les deux faces intérieure (56) et extérieure (53), le patin d'application (5) étant réalisé en zamak, et recouvert d'une couche protectrice barrière en matériau inerte, tel que du nickel et/ou du chrome, au moins au niveau du passage de distribution (50) et de la surface d'application (53), la tête comprenant en outre un corps en matière plastique (4) définissant une section de raccordement (4a) à l'unité de distribution (2), une section d'application (4c) en prise avec la face intérieure (56) du patin d'application (5), et une section de liaison (4b) reliant la section de raccordement (4a) à la section d'application (4c), le corps (4) définissant un canal de distribution (43) qui communique avec le passage de distribution (50) du côté de la face intérieure (56),
**caractérisée en ce que** le patin d'application (5) présente l'épaisseur minimum au niveau du passage de distribution (50), ladite épaisseur est de l'ordre de 1 à 4 mm.

2. Tête selon la revendication 1, dans lequel le patin d'application (5) comprend une zone périphérique d'épaisseur accrue et une zone centrale d'épaisseur réduite, le passage de distribution (50) étant formé au niveau de la zone d'épaisseur réduite.

3. Tête selon l'une quelconque des revendications précédentes, dans laquelle la face intérieure (56) présente une concavité supérieure à la convexité de la face extérieure (53).

4. Tête selon l'une quelconque des revendications précédentes, dans laquelle la section de liaison (4b) est souple de sorte que le patin d'application (5) est déplaçable par rapport à la section de raccordement (4a).

5. Tête selon l'une quelconque des revendications précédentes, dans laquelle la section d'application (4c) est surmoulée sur le patin d'application (5).

6. Tête selon l'une quelconque des revendications précédentes, dans laquelle la section d'application (4c) est en contact de la majeure partie de la face intérieure (56) du patin d'application (5).

7. Tête selon l'une quelconque des revendications précédentes, dans laquelle le canal de distribution (43) comprend une extrémité aval qui est directement adjacent au passage de distribution (50).

8. Tête selon l'une quelconque des revendications précédentes, dans laquelle le canal de distribution (43) définit une cavité sensiblement sphérique (46) immédiatement en amont du passage de distribution (50).

## Patentansprüche

1. Abgabe- und Applikationskopf (3), der dazu bestimmt ist, in Verbindung mit einer Abgabevorrichtung (2), wie zum Beispiel einer Pumpe, einem Ventil oder einer elastischen Tube verwendet zu werden, wobei der Kopf einen Applikationsgleiter (5) umfasst, die er eine äußere Applikationsoberfläche (53) aufweist, die dazu bestimmt ist, mit der Haut in Berührung zu kommen, wobei der Gleiter (5) im Wesentlichen aus einem Wärme abführenden Material hergestellt ist, um sich bei der Berührung mit der Haut kühl anzufühlen, wobei der Gleiter (5) ausgehend von einer Innenfläche (56) eine gewisse Dicke zur äußeren Oberfläche (53) hin aufweist und wobei der Gleiter (5) von einem Abgabekanal (50) durchzogen ist, der eine Verbindung zwischen der Innenfläche (56) und der äußeren Oberfläche (53) herstellt, wobei der Applikationsgleiter (5) aus Zamak hergestellt ist und zumindest im Bereich des Abgabekanals (50) und der Applikationsoberfläche (53)mit einer Barriere-Schutzschicht aus einem inerten Material wie zum Beispiel Nickel und/oder Chrom bedeckt ist, wobei der Kopf weiterhin einen Körper (4) aus Kunststoffmaterial aufweist, der einen Anschlussabschnitt (4a) zum Anschließen an die Abgabeeinheit (2), einen Applikationsabschnitt (4c) in Eingriff mit der Innenfläche (56) des Applikationsgleiters (5) und einen Verbindungsabschnitt (4b) umfasst, der den Anschlussabschnitt (4a) mit dem Applikationsabschnitt (4c) verbindet, wobei der Körper (4) einen Zuführkanal (43) umschließt, der mit dem Abgabekanal (50) auf der Seite der Innenfläche (56) in Verbindung steht,
**dadurch gekennzeichnet, dass** der Applikationsgleiter (5) im Bereich des Abgabekanals (50) eine minimale Dicke aufweist, die in der Größenordnung von 1 mm bis 4 mm liegt.

2. Kopf nach Anspruch 1, bei welchem der Applikationsgleiter (5) eine periphere Zone mit vergrößerter Dicke und eine zentrale Zone mit verringerter Dicke aufweist, wobei der Abgabekanal (50) im Bereich der Zone mit verringerter Dicke ausgebildet ist.

3. Kopf nach einem der vorhergehenden Ansprüche, bei dem die Innenfläche (56) einen oberen konkaven Bereich aufweist, der zur äußeren Oberfläche (53) hin konvex ist.

4. Kopf nach einem der vorhergehenden Ansprüche, bei dem der Verbindungsabschnitt (4b) in der Weise elastisch ist, dass der Applikationsgleiter (5) bezüglich des Anschlussabschnittes (4a) beweglich ist.

5. Kopf nach einem der vorhergehenden Ansprüche, bei dem der Applikationsabschnitt (4c) auf den Applikationsgleiter (5) aufgeformt ist.

6. Kopf nach einem der vorhergehenden Ansprüche, bei dem der Applikationsabschnitt (4c) mit dem Hauptteil der Innenfläche (56) des Applikationsgleiters (5) in Berührung steht.

7. Kopf nach einem der vorhergehenden Ansprüche, bei dem der Zuführkanal (43) ein stromabwärts liegendes Ende aufweist, das sich unmittelbar an den Abgabekanal (50) anschließt.

8. Kopf nach einem der vorhergehenden Ansprüche, bei dem der Zuführkanal (43) strömungsmäßig unmittelbar vor dem Abgabekanal (50) einen im Wesentlichen kugeligen Hohlraum (46) bildet.

## Claims

1. A dispenser and applicator head (3) for associating with a dispenser unit (2), such as a pump, a valve, or a squeezable tube, the head including an applicator pad (5) defining an outer applicator face (53) for coming into contact with the skin, the pad (5) being made essentially of a heat-transfer material so as to impart a cold sensation on contact with the skin, the pad (5) defining a thickness from an inner face (56) to the outer face (53), the pad (5) having a dispenser passage (50) passing through it, which passage puts the inner face (56) into communication with the outer face (53), the applicator pad (5) being made of zamak, and being covered with a protective barrier layer made of inert material, such as nickel and/or chromium, at least at the dispenser passage (50) and the applicator surface (53), the head further including a body (4) made of plastics material, said body defining a connection section (4a) for connecting to the dispenser unit (2), an application section (4c) in engagement with the inner face (56) of the applicator pad (5), and an interconnection section (4b) connecting the connection section (4a) to the application section (4c), the body (4) defining a dispenser channel (43) that communicates with the dispenser passage (50) beside the inner face(56),
the head being **characterized in that** the applicator pad (5) presents the minimum thickness at the dispenser passage (50), said thickness lying in the range about 1 mm to 4 mm.

2. A head according to claim 1, wherein the applicator pad (5) includes a peripheral zone of greater thickness and a central zone of smaller thickness, the dispenser passage (50) being formed through the zone of smaller thickness.

3. A head according to any preceding claim, wherein the inner face (56) presents concavity that is greater than the convexity of the outer face (53).

4. A head according to any preceding claim, wherein the interconnection section (4b) is flexible, such that the applicator pad (5) is movable relative to the connection section (4a).

5. A head according to any preceding claim, wherein the application section (4c) is overmolded on the applicator pad (5).

6. A head according to any preceding claim, wherein the application section (4c) is in contact with the major fraction of the inner face (56) of the applicator pad (5).

7. A head according to any preceding claim, wherein the dispenser channel (43) includes a downstream end that is directly adjacent to the dispenser passage (50).

8. A head according to any preceding claim, wherein the dispenser channel (43) defines a cavity (46) that is substantially spherical, immediately upstream from the dispenser passage (50).
